(19) Europäisches Patentamt
European Patent Office
Office européen des brevets

(11) Veröffentlichungsnummer: **0 447 949 A1**

(12) **EUROPÄISCHE PATENTANMELDUNG**

(21) Anmeldenummer: **91103888.3**

(22) Anmeldetag: **14.03.91**

(51) Int. Cl.[5]: **G01N 31/22**, G01N 21/64, A61B 5/00

(30) Priorität: **22.03.90 CH 955/90**

(43) Veröffentlichungstag der Anmeldung: **25.09.91 Patentblatt 91/39**

(84) Benannte Vertragsstaaten: **CH DE FR GB IT LI NL**

(71) Anmelder: **F. HOFFMANN-LA ROCHE AG**
**Postfach 3255**
**CH-4002 Basel(CH)**

(72) Erfinder: **Barner, Richard, Dr.**
**Traubenweg 16**
**CH-4108 Witterswil(CH)**

Erfinder: **Huber, Walter, Dr.**
**Ziegelhofweg 62**
**CH-4303 Kaiseraugst(CH)**
Erfinder: **Hübscher, Josef**
**Säspelstrasse 1**
**CH-4208 Nunningen(CH)**
Erfinder: **Müller, Francis**
**Beim Wasserturm 32**
**CH-4059 Basel(CH)**

(74) Vertreter: **Kloter, Rudolf et al**
**Postfach 3255 Grenzacherstrasse 124**
**CH-4002 Basel(CH)**

(54) **Optischer Sauerstoffsensor.**

(57) Die Erfindung betrifft einen optischen Sauerstoffsensor, der auf der Uebertragung elektromagnetischer Energie von einem Donor auf einen Akzeptor beruht. Der Donor ist ein Pyren-Derivat der allgemeinen Formel

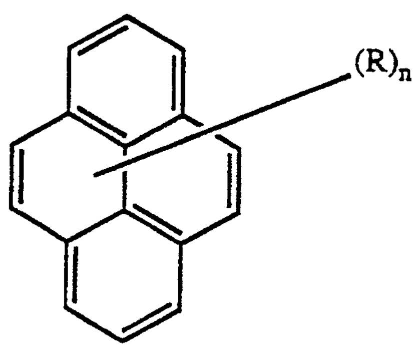

worin R eine gegebenenfalls mit einer Hydroxy-, Amino-, oder einer Gruppe der Formel -NH(1-8C-Alkyl)-substituierte Alkylgruppe mit bis zu 30 C-Atomen bedeutet, eine Gruppe $-CH_2-OR$ bedeutet, wobei R die obengenannte Bedeutung hat, oder eine Gruppe -OR bedeutet, wobei R die obengenannte Bedeutung hat, oder eine Gruppe R'-O-R'' bedeutet, wobei R' eine Alkylengruppe bedeutet, R'' die obige Bedeutung von R hat und R' und R'' zusammen nicht mehr als 30 C-Atome aufweisen und n eine ganze Zahl von 1-10 bedeutet.

Der Akzeptor ist ein Anthrazen-Derivat der allgemeinen Formel

EP 0 447 949 A1

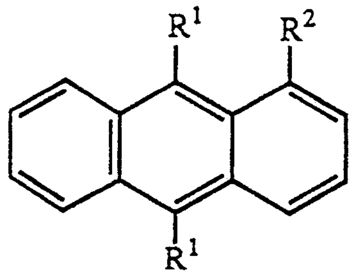

oder

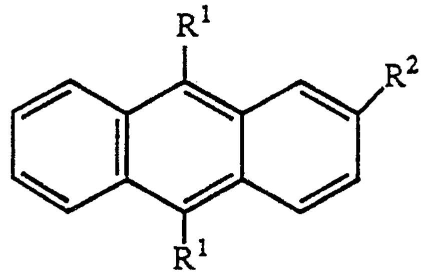

worin $R^1$ Wasserstoff, Alkyl oder Alkoxy bedeutet und $R^2$ eine Acylaminogruppe bedeutet, die gegebenenfalls eine Verankerungsgruppe, wie z.B. eine Hydroxygruppe, enthalten kann, ist.

Ein bevorzugter Donor ist 1-(11-Hydroxyundecyloxymethyl)pyren und ein bevorzugter Akzeptor 2-(4-Hydroxybutanoylamino)-9,10-dimethoxyanthracen.

Die vorliegende Erfindung betrifft einen optischen Sauerstoffsensor. Derartige Sensoren bestehen vielfach aus einem lichtleitenden Medium, auf das eine sauerstoffsensitive Schicht aufgebracht wurde. Solche Sensoren können einem Patienten implantiert werden und dienen insbesondere zur Ueberwachung des Sauerstoffgehaltes bei Intensivpatienten.

Solche optische Sauerstoff($O_2$)sensoren sind in der Patentliteratur und der Literatur beschrieben, vgl. z.B. Europäische Patentanmeldung, Veröffentlichungs-Nr. 91,390, Europäische Patentanmeldung, Veröffentlichungs-Nr. 252,578 sowie Journal of Optical Sensors, 1986, Vol. 1, No. 1, pp. 43-67.

Zum Beispiel kann bei diesen Sensoren Licht über eine Optik und einen dichroitischen Spiegel in eine Glasfaser (lichtleitendes Medium) eingekoppelt werden. Dies regt am Ende der Glasfaser in der $O_2$-sensitiven Schicht den $O_2$-Indikator an. Das Fluoreszenzlicht des $O_2$-Indikatorswird in der Glasfaser gesammelt und durch diese wieder an den Eingang der Faser zurückgeführt. Dieses Fluoreszenzlicht wird über den dichroitischen Spiegel, Interferenzfilter und Linsen dem Photomultiplier zugeführt. Aus dem Signal des Photomultipliers werden unter zu Hilfenahme einer Auswerteelektronik Aussagen über Fluoreszenzintensität bzw. Fluoreszenzlebensdauer möglich. Die beiden Grössen können in Bezug gesetzt werden zu der $O_2$-Konzentration in der Nähe der $O_2$-sensitiven Schicht.

Das $O_2$-sensitive Element eines $pO_2$-Sensors kann auf zwei grundsätzlich verschiedene Arten ausgebildet sein.

Die $O_2$-sensitive Schicht kann auf das Faserende aufgebracht werden. Das Anregungslicht tritt aus dem Faserende aus und regt die Fluoreszenz der $O_2$-Indikatoren an. Da das abgestrahlte Fluoreszenzlicht isotrop verteilt ist, wird ein Teil wieder in die Faser eingekoppelt und durch die Faser dem Photomultiplier zugeführt.

Die sensitive Schicht kann über eine gewisse Strecke die Auskleidung der Faser ersetzen. Das Anregungslicht wird in dieser Ausbildung des sensitiven Elementes über den gesamten Bereich der sensitiven Schicht im Kern der Glasfiber geführt. Die Wechselwirkung der $O_2$-Indikatoren mit dem Anregungslicht erfolgt über das sogenannte "evaneszente" Feld (Teil des elektromagnetischen Feldes, welches bei Totalreflexion in das optisch dünnere Medium eindringt). Von dieser Anregung sind nur Indikatoren betroffen, welche sich innerhalb der Reichweite dieses evaneszenten Feldes befinden. Die Einkopplung des Fluoreszenzlichtes erfolgt hier über das "evaneszente" Feld.

Die $O_2$-sensitive Schicht besteht aus $O_2$-Indikatormolekülen. Die $O_2$-Indikatormoleküle werden in Polymeren eingebracht, welche unter dem Sammelbegriff Silikone zusammengefasst sind. Der einfachste Vertreter dieser Klasse ist Polydimethylsiloxan. Eine Vielzahl von Gruppen (z.B. Alkyl, Phenyl, Trifluoropropyl, Vinyl, Wasserstoff etc.) kann Methyl als Substituent an Silizium ersetzen. Darüberhinaus könne einzelne repetierende Einheiten durch Verzweigungspunkte ersetzt sein. Diese erlauben eine Quervernetzung einzelner linearer Ketten. Die Folgen dieser Quervernetzung sind hochmolekulare Silikongummis. Silikon wird aufgrund seiner hohen $O_2$-Permeabilität bzw. $O_2$-Löslichkeit als Trägermaterial für die $O_2$-Indikatoren gewählt.

Die Darstellung der Silikonmatrix erfolgt z.B. ausgehend von sogenannten RTV's (room-temperature-vulcanizing systems) welche unter dem Einfluss der Luftfeuchtigkeit aushärten. Sie enthalten als reaktive Gruppen in allgemeinen Acyloxi (= SiOAc), Amine (= $SiNR_2$), Oxime (= $Si\text{-}O\text{-}N{=}CR_2$) oder Alkyloxy (= SiOR). Diese RTV's werden in einem org. Lösungsmittel gelöst, welches die Indikatormoleküle enthält. Diese Lösung wird in einer dünnen Schicht auf das lichtleitende Substrat aufgebracht. In Kontakt mit Umgebungsfeuchtigkeit erfolgt das Aushärten des Polymers.

Erfindungsgemäss besteht der $O_2$-Indikator nicht aus einem einzigen Fluorophor, sondern aus einem Energie-Donor-Akzeptor-Paar, wie es z.B. in Applied Spectroscopy (Vol. 42, No. 6, 1988, 1009-1011) für die Kombination von Pyren und Perylen beschrieben ist. Es wurde nun gefunden, dass sich die Kombination von Pyren-1-Aminoanthracen sowie Pyren-2-Amino-anthracen besser als $O_2$-Indikatoren eignen als die Kombination Pyren-Perylen. Dies wird zurückgeführt auf eine bessere Ueberlappung des Emissionsspektrums von Pyren mit dem Absorptionsspektrum von 1- bzw. 2-Aminoanthracen. Insbesondere ist die molare Extinktion der Aminoanthracenderivate im Wellenlängenintervall maximaler Pyrenabsorption verschwindend klein. Dadurch ist sichergestellt, dass alles durch das Aminoanthracen emitierte Licht auf eine indirekte Anregung des Aminoanthracens durch das Pyren zurückzuführen ist.

Die Kombination Pyren-Aminoanthracen eignet sich deshalb aus folgenden Gründen ausgezeichnet für einen $O_2$-Nachweis: Der angeregte Zustand des Pyrens (Anregungswellenlänge: $320 < \lambda < 340$ nm) wird effizient durch Sauerstoff gequencht. Das Emissionsspektrum von Pyren (Energie-Donor) überlappt gut mit den Absorptionsspektren der Energie-Akzeptoren (1-Aminoanthracen, 2-Aminoanthracen). Die Energie-Uebertragungsrate ist direkt abhängig vom Löschen des angeregten Zustandes von Pyren durch $O_2$, sodass das Emissionsverhalten der Aminoanthracene (Fluoreszenzlebensdauer, Fluoreszenzintensität) des Aminoanthracens (Emissionswellenlänge: $440 < \lambda < 500$ nm) in Bezug gesetzt werden kann zur $O_2$-

Konzentration innerhalb der $O_2$-sensitiven Schicht. Anregungswellenlänge ($320 < \lambda < 340$ nm) und Emissionswellenlänge ($440 < \lambda < 500$ nm) des $O_2$-Indikators (= Donor-Akzeptor-Paar) sind also um mehr als 100 nm separiert, sodass eine Fluoreszenzmessung kaum durch Streulicht bzw. Eigenfluoreszenz der Messeinrichtung beeinflusst wird.

Die vorliegende Erfindung betrifft somit ein optischen Sauerstoffsensor basierend auf einer Uebertragung elektromagnetischer Energie von einem Donor auf einen Akzeptor, welcher dadurch gekennzeichnet ist, dass der Donor ein Pyrenderivat der allgemeinen Formel

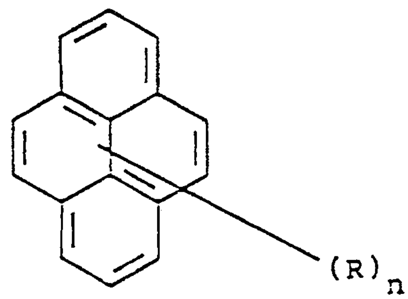

I

worin R eine gegebenenfalls mit einer Hydroxy-, Amino-, oder einer Gruppe der Formel -NH(1-8C Alkyl)-substituierte Alkylgruppe mit bis zu 30 C-Atomen bedeutet, eine Gruppe $-CH_2$-OR Gruppe oder eine Gruppe -OR bedeutet, wobei R die obengenannte Bedeutung hat, oder eine Gruppe R'-O-R" bedeutet, wobei R' eine Alkylengruppe bedeutet, R" die obige Bedeutung von R hat und R' und R" zusammen nicht mehr als 30 C-Atome aufweisen und n eine ganze Zahl von 1-10 bedeutet,
ist, und dass der Akzeptor ein Anthracenderivat der allgemeinen Formel

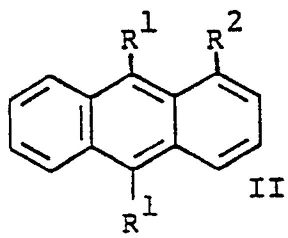

II

oder

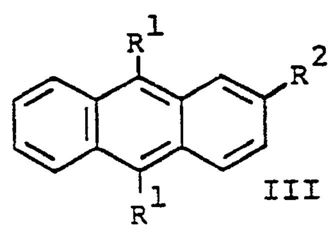

III

ist, wobei $R^1$ Wasserstoff, Alkyl oder Alkoxy bedeutet und $R^2$ eine Acylaminogruppe bedeutet, die gegebenenfalls eine Verankerungsgruppe, wie z.B. eine Hydroxylgruppe enthalten kann.

Als Donoren kommen insbesondere Dioctadecylpyren, Tetraoctadecylpyren, 1-(Methoxymethyl)pyren, 1-(Octadecyloxymethyl)pyren, 1-Hydroxymethylpyren, 1-(11-Hydroxyundecyl)-pyren, 1-(11-Hydroxyundecyloxymethyl)pyren sowie 1-(2-Aminoäthyloxymethyl)pyren in Betracht.

Soweit die Pyrenderivate der allgemeinen Formel I eine substituierte Alkylgruppe R aufweisen, werden diese Indikatormoleküle gleichzeitig mit der Aushärtung der Silikonmatrix an das Polymergerüst kovalent gebunden. Diese kovalente Bindung beruht auf der Reaktion zwischen den reaktiven Gruppen des Prepolymers sowie der reaktiven Gruppen der Indikatormoleküle. Dasselbe trifft zu, wenn das Anthracenderivat der allgemeinen Formel II oder III eine substituierte Acylaminogruppe $R^2$ aufweist. Die Pyren- bzw. Anthracenderivate liegen bevorzugt in eine Konzentration von $10^{-4}$-$10^{-2}$ Mol/Liter vor.

Als Akzeptoren kommen insbesondere 1-Palmitoylaminoanthracen, 1-(3-Hydroxypropionylamino)-anthracen, 1-[3-(2-Hydroxy-1,1-dimethyläthyl-aminocarbonyl) propionylamino]anthracen, 1-Acetylamino-9,10-dimethoxyanthracen, 2-Acetylamino-9,11-dimethoxyanthracen, 2-Palmitoylamino-9,10-dimethoxyanthracen sowie 2-(3-Hydroxypropionylamino)-9,10-dimethoxyanthracen in Betracht.

Gemäss vorliegender Erfindung dienen als Energieakzeptormoleküle Derivate des 1-Aminoanthracens und des 2-Aminoanthracens (vergl. Formelschema). Dabei wurden die Aminoanthracene dahingehend modifiziert, dass a) eine Langzeitstabilität des Akzeptors garantiert werden kann, b) die Derivate des Aminoanthracens in genügend hoher Konzentration in den Silikonpolymeren löslich sind und c) gewisse Derivate kovalent mit dem Silikongrundgerüst verknüpft werden können, sodass ein Auswaschen des Indikators vermieden werden kann.

Die Verbindungen der Formel I können in an sich bekannter Weise durch Alkylierung von Pyren mit den entsprechend substituierten Alkylhalogeniden in einer Friedel Crafts Reaktion mit einem Katalysator, wie z.B. Aluminiumchlorid, in einem inerten Lösungsmittel, wie Tetrachlorkohlenstoff, Methylenchlorid oder in desaktivierten flüssigen Aromaten, wie Nitrobenzol, Chlorobenzol, vorzugsweise Methylenchlorid bei 0-30° C, bevorzugt Raumtemperaur, hergestellt werden. Die Einführung der Alkylgruppen erfolgt entsprechend dem für den betreffenden Aromaten charakteristischen Substitutionsmuster der elektrophilen Substitution. Nach üblicher Aufarbeitung mit Eiswasser und Methylenchlorid können die Alkylierungsprodukte durch Chromatographie getrennt werden.

Die Verbindungen der Formel I, worin R $CH_2$-OR, R'-O-R" oder OR bedeuten, können durch Ueberführung der entsprechenden Hydroxyverbindung in das Alkoholat durch Deprotonierung mit Natriumhydrid bei 0° C bis Raumtemperatur in einem inerten für die anschliessende Alkylierung günstigen Lösungsmittel wie Tetrahydrofuran oder N,N-Dialkylformamid und Umsetzung (nach abgeklungener $H_2$-Entwicklung) mit dem entsprechenden substituierten Alkylhalogenid bei Raumtemperatur in die entsprechenden substituierten Alkoxy-Verbindungen überführt werden.

Die Verbindungen der Formel II und III können gemäss beiliegendem Schema hergestellt werden.

Die Verbindungen der Formeln II und III, worin $R^1$ = H und $R^2$ = Acylamino bedeutet, können in an sich bekannter Weise aus dem entsprechenden Amin mit dem entsprechenden aktiven Carbonsäurederivat (z.B. Säurechlorid, Säureanhydrid oder ähnlichem) in den dafür üblichen Lösungsmitteln hergestellt werden. Die Verbindungen der Formeln II und III, worin $R^1$ = H und $R^2$ eine substituierte Alkylgruppe enthält, können gemäss vorher beschriebener Methode mit einer entsprechenden aktivierten Dicarbonsäure umgesetzt werden und die so erhaltene Monocarbonsäure a) mit einem Hydrid, wie z.B. Borhydride, nach bekannter Weise in den dafür üblichen Lösungsmitteln zum Alkohol reduziert, oder b) nach der oben beschriebenen Methode die Carbonsäuregruppe aktiviert und anschliessend mit dem entsprechenden Aminoalkohol umgesetzt werden. Verbindungen der Formeln II und III, worin $R^1$ eine Alkoxygruppe und $R^2$ eine Acylaminogruppe bedeutet, werden bevorzugt aus den entsprechend acylaminosubstituierten Chinonen hergestellt. Dazu wird die Chinongruppe zuerst nach den dafür üblichen Bedingungen reduziert (katalytisch mit Wasserstoff oder mit Dithioniten) und anschliessend in ebenfalls bekannter Weise unter Verwendung einer Base, wie beispielsweise Alkalihydroxyden oder -carbonaten, mit einem entsprechenden Alkyl-halogenid oder -sulfonat bevorzugt unter Verwendung eines quaternären Ammoniumsalzes als Phasentransferkatalysator in den dafür üblichen Lösungsmitteln umgesetzt. Verbindungen der Formel II und III, worin $R^1$ eine Alkoxy- und $R^2$ eine substituierte oder unsubstituierte Acylaminogruppe bedeutet, werden aus den entsprechend Acylamino-dialkoxy-substituierten Derivaten durch Verseifung der Amid-Gruppe in üblicher Weise über das so erhaltene Amin nach der oben beschriebenen Methode mit dem entsprechend aktivierten Dicarbonsäure- oder Monocarbonsäure-Derivat umgesetzt und gegebenenfalls entsprechend den beiden Möglichkeiten (a, b) weiterverarbeitet.

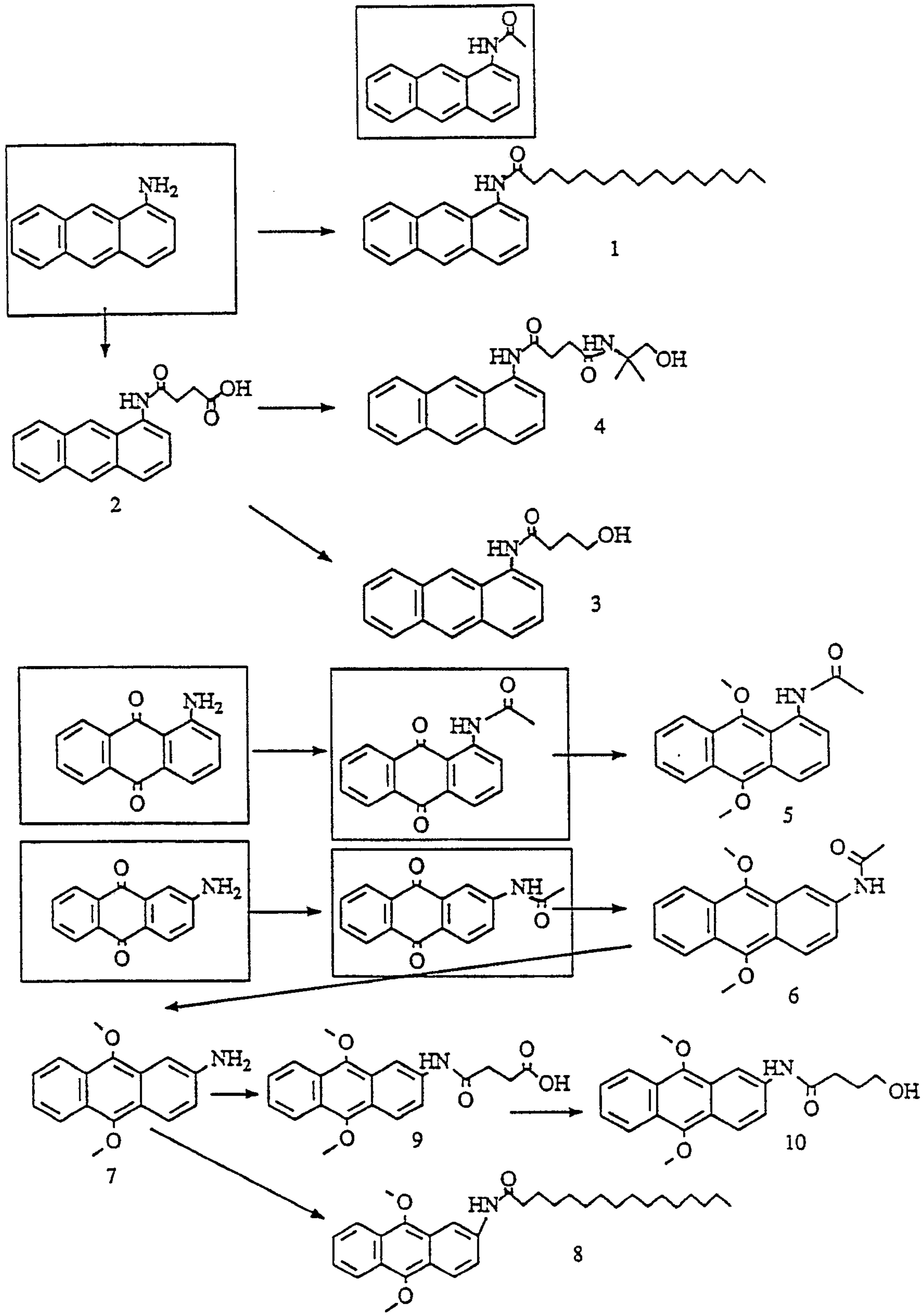

1

4

2

3

5

6

7

9

10

8

Die nachfolgenden Beispiele erläutern die Erfindung:

Beispiel 1

Es werden 2 g (10 mMol) Pyren in 10 ml trockenem Methylenchlorid zu einer Suspension von 2,5 g (10

mMol) 11-Bromundecanol und 20 g (160 mMol) Aluminiumchlorid in 10 ml Methylenchlorid zugetropft, worauf während 16 Stunden bei Raumtemperatur gerührt wird. Es wird mit Eiswasser hydrolysiert, mit Methylenchlorid extrahiert und eingeengt. Es wird mit Methylenchlorid chromatographiert. Es werden dabei 1,9 g (50%) 1-(11-Hydroxyundecyl)-pyren erhalten. HPLC (0,5% EtOAc/Hexan) $t_R$ = 3,4 Minuten (Ed = 2,1 Minuten) erhalten.

Beispiel 2

Es werden 1,86 g (8 mMol) 1-Hydroxymethylpyren in 20 ml trockenem DMF gelöst und mit 300 mg (9 mMol) Natriumhydrid (80 in Mineralöl) versetzt und 30 Minuten bei Raumtemperatur gerührt. Dann werden 2 g (8 mMol) 1-Brom-11-undecanol zugetropft und 3 Stunden bei Raumtemperatur gerührt. Es wird mit Eiswasser versetzt, mit Toluol extrahiert und die organische Phase eingeengt und an Kieselgel chromatographiert. Es werden 3,4 g (99%) 1-(11-Hydroxyundecyloxymethyl)pyren vom Schmelzpunkt 62-64°C erhalten und im MS und NMR charakterisiert. DC (Kieselgel/Toluol) Rf (Edukt) = 0,1 Rf (Produkt) = 0,11 und HPLC (RP18 125-4/MeCN) tR (Edukt) = 2,3 Minuten tR (Produkt) = 6,5 Minuten.

Beispiel 3

Es wurden 20 g (100 mMol) Pyren als Lösung in 100 ml trockenem Methylenchlorid zugetropft zu einer Suspension von 35 g (105 mMol) 1-Bromoctadecan und 5 g (40 mMol) Aluminiumchlorid in 10 ml Methylenchlorid und während 5 Stunden bei Raumtemperatur gerührt. Es wurde mit Eiswasser hydrolysiert, mit Hexan extrahiert und eingeengt. Das Rohprodukt wurde an Kieselgel mit Hexan chromatographiert. Es wurden dabei 16 g (22,6%) Dioctadecylpyren und 19 g (15,7%) Tetraoctadecylpyren (als Isomerengemisch neben Edukt und anderen Alkylierungsprodukten) als gelbe Oele erhalten. Die Produkte wurden im IR, NMR und MS und Mikroanalyse charakterisiert.
DC (Kieselgel/Hexan) Rf (Edukt) = 0,4 Rf (Dioctadecylpyren/Tetraoctadecylpyren) = 0,7/0,8
Mol(C16H10) = 202,26
Mol(C52H82) = 707,23
Mol(C88H154) = 1212,21.

Beispiel 4

Eine Lösung von 186 mg (0,8 mMol) 2-(Hydroxymethyl)pyren in trockenem N,N-Dimethylformamid (DMF) wurde bei 0°C mit 100 mg einer 55-60%-igen Suspension von Natriumhydrid in Mineralöl (ca. 2 mMol) Natriumhydrid) unter Rühren versetzt und nach abgeklungener Wasserstoffentwicklung noch 30 Minuten bei Raumtemperatur gerührt. Zu der erhaltenen Lösung hat man eine Lösung von 270 mg (0,8 mMol) Octadecylbromid in 1 ml trockenem DMF unter Rühren zugetropft und anschliessend das Reaktionsgemisch noch über Nacht gerührt (Raumtemperatur). Durch Filtration über "Hyflo" wurden die Salze abgetrennt und das Filtrat direkt an Kieselgel chromatographiert, wobei 363 mg (85%) 1-(11-Octadecyloxymethyl)pyren kristallin erhalten wurden; Schmelzpunkt = 67-69°C.
Die Verbindung wurde im Massenspektrum charakterisiert (M+ = 484)
DC (Kieselgel/Toluol) Rf = 0,8 Rf (Hydroxymethylpyren) = 0,1 Mol (C35H480) = 484,73.

Beispiel 5

Es werden 6,20 g (27 mMol) 1-Hydroxymethylpyren in 10 ml trockenem DMF gelöst und mit 760 mg (27 mMol) Natriumhydrid (80% in Mineralöl) versetzt und 30 Minuten bei Raumtemperatur gerührt. Dann werden 16 g (52 mMol) 1,11-Dibromundecan zugetropft und 16 Stunden bei Raumtemperatur gerührt. Es wird mit Eiswasser versetzt, mit Toluol extrahiert und die organische Phase durch Kieselgel filtriert und eingeengt. Es wird mit Toluol an Kieselgel chromatographiert. Man erhält 8,54 g (63%) 11-Brom-1-(pyrenylmethyloxy)undecan vom Schmelzpunkt 45-47°C das im MS und NMR charakterisiert wird.
DC (Kieselgel/Toluol) Rf (Edukte) = 0,1 und 0,9 Rf (Produkt) = 0,8 Rf (Produkt) = 0,5 und HPLC (RP18 125-4/MeCN) tR (Edukt) = 2,3 Minuten tR (Produkt) = 4,6 Minuten.

Beispiel 6 1 g (ca. 2 mMol) 11-Brom-2-(1-Pyrenmethyloxy)undecan werden in 10 ml THF gelöst und mit 3 g Phthalimid-Kalium über Nacht bei Raumtemperatur gerührt. Es wird eingeengt und anschliessend mit Wasser und Methylenchlorid extrahiert. Nach dem Trocknen wird die Methylenchloridlösung mit 10 ml Hydrazinhydrat versetzt und über Nacht gerührt. Es wird eingeengt, in Aethanol suspendiert, filtriert und das

Filtrat wird eingeengt. Man erhält 0,8 g (ca. 100%) 11-Amino-1-(1-pyrenylmethoxy)undecan. Zur Reinigung für die Elementaranalyse wird aus 1N Salzsäure umkristallisiert. Man erhält 0,5 g des entsprechenden Hydrochlorides.

Beispiel 7

500 mg 1-Hydroxymethylpyren werden in 10 ml trockenem Dimethylformamid gelöst und bei -20° 100 mg Natriumhydrid (55% in Mineralöl) zugegebem. Es wird 30 Minuten gerührt und dann 0,5 ml 2-Chloräthylamin zugetropft. Es wird über Nacht bei Raumtemperatur nachgerührt, anschliessend erst mit Aethanol und dann mit Wasser versetzt. Es wird eingeengt und aus Chloroform/Hexan umgefällt. Man erhält 410 mg 1-(2-Aminoäthyloxymethyl)pyren.

Beispiel 8

In 20 ml Pyridin werden 1,6 g 1-Aminoanthracen gelöst. Dann werden 5 ml Palmitoylchlorid zugetropft. Es wird über Nacht bei Raumtemperatur gerührt. Es werden 50 ml Natriumbicarbonatlösung (10%) zugetropft und 1 Stunde nachgerührt. Es wird filtriert, mit Wasser gewaschen und der Rückstand wird bei 40° C im Wasserstrahlvakuum getrocknet. Man erhält 4,09 g 1-Palmitoylaminoanthracen vom Schmelzpunkt 162° C.

Beispiel 9

Es werden 0,50 g 1-Aminoanthracen in 10 ml Pyridin gelöst und dann 2 g Bernsteinsäureanhydrid zugegeben. Es wird über Nacht bei Raumtemperatur gerührt. Dann werden 10 ml Wasser zugetropft und 1 Stunde gerührt. Dann werden 100 ml 1N Salzsäure unter Rühren zugegeben und anschliessend filtriert. Der Rückstand wird mit Wasser gewaschen und getrocknet. Man erhält 0`52 g 1-(3-Carboxylpropionylamino)-anthracen.

Beispiel 10

Es werden 0,5 g 1-(3-Carboxypropionylamino)-anthracen gelöst in 10 ml Tetrahydrofuran und tropfenweise versetzt mit 0,5 ml Borandimethylsulfid. Es wird 5 Stunden bei Raumtemperatur gerührt und anschliessend 10 ml Wasser zugetropft. Es wird filtriert und mit Wasser gewaschen. Man erhält 0,42 g 1-(4-Hydroxybutanoylamino)-anthracen.

Beispiel 11

Es werden 0,1 g 1-(3-Carboxypropionylamino)-anthracen gelöst in 1 ml Thionylchlorid und 1 Stunde bei Rückfluss gerührt. Es wird eingeengt und anschliessend mit 1 ml 2-Aminoisobutanol versetzt. Es wird über Nacht bei Raumtemperatur gerührt und anschliessend 10 ml Wasser zugetropft. Dann werden 10 ml 1N Salzsäure zugegeben, filtriert und mit Wasser gewaschen. Man erhält 0,11 g 1-[3-(2-Hydroxy-1,1-dimethyläthylaminocarbonyl) -propionylamino]-anthracen.

Beispiel 12

Es werden 2,6 g 1-Acetylaminoanthrachinon in 50 ml Sodalösung 5% und 5 g Natriumdithionit gelöst. Dann werden 0,1 g Tetrabutylammoniumhydrogensulfat zugegeben und anschliessend 3 ml Dimethylsulfat zugetropft. Es wird über Nacht bei Rückfluss gerührt. Es wird mit Methylenchlorid extrahiert, mit Natriumsulfat getrocknet und eingeengt. Die Chromatographie an Kieselgel mit Essigester ergab 0,3 g 1-Acetylamino-9,10-dimethoxyanthracen.

Beispiel 13

Es werden 2,6 g 2-Acetylaminoanthrachinon in 50 ml Sodalösung 5% und 5 g Natriumdithionit gelöst. Dann werden 0,1 g Tetrabutylammoniumhydrogensulfat zugegeben und anschliessend 3 ml Dimethylsulfat zugetropft. Es wird über Nacht bei Rückfluss gerührt. Es wird mit Methylenchlorid extrahiert, mit Natriumsulfat getrocknet und eingeengt. Die Chromatographie an Kieselgel mit Essigester ergibt 1,8 g 2-Acethylamino-9,10-dimethoxyanthracen.

Beispiel 14

Es werden 1,5 g 2-Acetylamino-9,10-dimethoxyanthracen in 100 ml 1N Natronlauge über Nacht bei Rückfluss gerührt. Es wird mit Methylenchlorid extrahiert, mit Natriumsulfat getrocknet und eingeengt. Es werden 1,3 g 2-Amino-9,10-dimethoxyanthracen erhalten.

Beispiel 15

In 5 ml Pyridin werden 0,5 g 2-Amino-9,10-dimethoxyanthracen gelöst. Dann werden 1 ml Palmitoylchlorid zugetropft. Es wird über Nacht bei Rückfluss gerührt. Es werden 50 ml Natriumbicarbonatlösung (10%) zugetropft und 1 Stunde nachgerührt. Es wird filtriert, mit Wasser gewaschen und der Rückstand wird bei 40°C im Wasserstrahlvakuum getrocknet. Man erhält 0,4 g 2-Palmitoylamino-9,10-dimethoxyanthracen.

Beispiel 16

Es werden 0,50 g 2-Amino-9,10-dimethoxyanthracen in 10 ml Pyridin gelöst und dann 2 g Bernsteinsäureanhydrid zugegeben. Es wird über Nacht bei Rückfluss gerührt. Dann werden 10 ml Wasser zugetropft und 1 Stunde bei Raumtemperatur gerührt. Dann werden 100 ml 1N Salzsäure unter Rühren zugegeben und anschliessend filtriert. Der Rückstand wird mit Wasser gewaschen und getrocknet. Man erhält 0,3 g 2-(3-Carboxypropionylamino)-9,10-dimethoxyanthracen.

Beispiel 17

Es werden 0,3 g 2-(3-Carboxypropionylamino)-9,10-dimethoxyanthracen gelöst in 10 ml Tetrahydrofuran und tropfenweise versetzt mit 0,3 ml Borandimethylsulfid. Es wird 5 Stunden bei Raumtemperatur gerührt und anschliessend 10 ml Wasser zugetropft. Es wird filtriert und mit Wasser gewaschen. Man erhält 0,2 g 2-(4-Hydroxybutanoylamino)-9,10-dimethoxyanthracen.

Beispiel 18

Herstellung einer $O_2$-sensitiven Schicht mit nicht kovalent gebundenen Indikatoren

1 g Elastosil E43 (Wacker Chemie) wird in 1 ml einer Toluollösung des Indikatorpaares 1-Palmitoylaminoanthracen und Dioctadecylpyren (Konzentrationen ca. $1*10^{-3}$ Mol/l) gelöst. Die resultierende zähflüssige Masse wird in gewünschter Schichtdicke auf das Glassubstrat aufgebracht und 24 Stunden ausgehärtet.

Beispiel 19

Herstellung einer $O_2$-sensitiven Schicht mit kovalent gebundenen Indikatoren

1 g Elastosil E43 (Wacker Chemie) wird in 1 ml einer Toluollösung der Indikatoren 1-(11-Hydroxyundecyloxymethyl)-pyren ($2*10^{-3}$ Mol/l) und 2-(4-Hydroxybutanoylamino)-9,10-dimethoxyanthracen ($1*10^{-3}$ Mol/l) gelöst und die zähflüssige Masse in gewünschter Schichtdicke auf das Glassubstrat aufgebracht. Nach dem Aushärten wird der nicht kovalent gebundene Indikator durch Waschen mit Methylenchlorid aus der Silikonmatrix herausgelöst.

**Patentansprüche**

**1.** Optischer Sauerstoffsensor basierend auf einer Uebertragung elektromagnetischer Energie von einem Donor auf einen Akzeptor, dadurch gekennzeichnet, dass der Donor ein Pyrenderivat der allgemeinen Formel

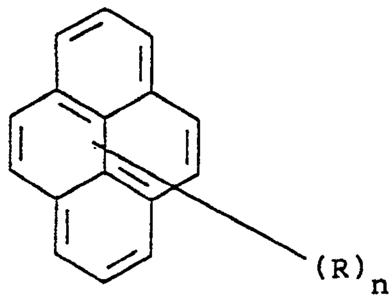

I

worin R eine gegebenenfalls mit einer Hydroxy-, Amino-, oder eine Gruppe der Formel -NH(1-8C Alkyl)-substituierte Alkylgruppe mit bis zu 30 C-Atomen bedeutet, eine Gruppe $-CH_2-OR$ Gruppe bedeutet, wobei R die obengenannte Bedeutung hat, oder eine Gruppe -OR bedeutet, wobei R die obengenannte Bedeutung hat, oder eine Gruppe R'-O-R" bedeutet, wobei R' eine Alkylengruppe bedeutet, R" die obige Bedeutung von R hat und R' und R" zusammen nicht mehr als 30 C-Atome aufweisen und n eine ganze Zahl von 1-10 bedeutet,
ist, und dass der Akzeptor ein Anthracenderivat der allgemeinen Formeln

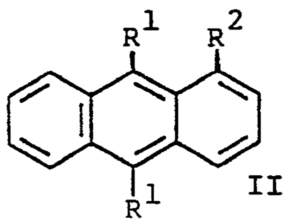

II

oder

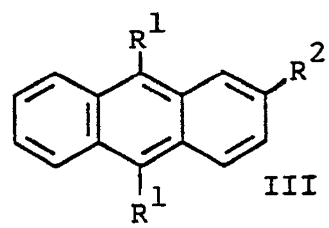

III

worin $R^1$ Wasserstoff, Alkyl oder Alkoxy bedeutet und $R^2$ eine Acylaminogruppe bedeutet, die gegebenenfalls eine Verankerungsgruppe, wie z.B. eine Hydroxygruppe enthalten kann,
ist.

2. Optischer Sauerstoffsensor gemäss Patentanspruch 1, dadurch gekennzeichnet, dass als Donor Dioctadecylpyren, Tetraoctadecylpyren, 1-(Methoxymethyl)pyren, 1-(Octadecyloxymethyl)pyren, 1-Hydroxymethylpyren, 1-(11-Hydroxyundecyl)pyren, 1-(11-Hydroxyundecyloxymethyl)pyren oder 1-(2-Aminoäthyloxymethyl)pyren verwendet wird.

3. Optischer Sauerstoffsensor gemäss Patentanspruch 1, dadurch gekennzeichnet, dass als Akzeptor 1-Palmitoylaminoanthracen, 1-(4-Hydroxybutanoylamino)anthracen, 1-[3-(2-Hydroxy-1,1-dimethyläthylaminocarbonyl) propionylamino]anthracen, 1-Acetylamino-9,10-dimethoxyanthracen, 2-Acetylamino-9,10-dimethoxyanthracen, 2-Palmitoylamino-9,10-dimethoxyanthracen oder 2-(4-Hydroxybutanoylamino)-9,10-dimethoxyanthracen verwendet wird.

4. Optischer Sauerstoffsensor, dadurch gekennzeichnet, dass als Donor Dioctadecylpyren und als Akzeptor 1-Palmitoylaminoanthracen verwendet wird.

5. Optischer Sauerstoffsensor, dadurch gekennzeichnet, dass als Donor 1-(11-Hydroxyundecyloxymethyl)-pyren und als Akzeptor 2-(4-Hydroxybutanoylamino)-9,10-dimethoxyanthracen verwendet wird.

Europäisches Patentamt

# EUROPÄISCHER RECHERCHENBERICHT

Nummer der Anmeldung

**EP 91 10 3888**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 381 026 (F. HOFFMANN-LA ROCHE AG)<br>* Das ganze Dokument *<br>– – – | 1-5 | G 01 N 31/22<br>G 01 N 21/64<br>A 61 B 5/00 |
| D,A | APPLIED SPECTROSCOPY, Band 42, Nr. 6, August 1988, Seiten 1009-1011, Baltimore, MD, US; A. SHARMA et al.: "Fiberoptic oxygen sensor based on fluorescence quenching and energy transfer"<br>* Das ganze Dokument *<br>– – – | 1-5 | |
| D,A | EP-A-0 091 390 (UNITED STATES OF AMERICA)<br>* Das ganze Dokument *<br>– – – – – | 1-5 | |
| | | | **RECHERCHIERTE SACHGEBIETE (Int. Cl.5)** |
| | | | G 01 N<br>C 07 C<br>A 61 B |

Der vorliegende Recherchenbericht wurde für alle Patentansprüche erstellt

| Recherchenort | Abschlußdatum der Recherche | Prüfer |
|---|---|---|
| Den Haag | 18 Juni 91 | DOEPFER K-P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument